**Europäisches Patentamt**

**European Patent Office**

**Office européen des brevets**

(19)

(11) Veröffentlichungsnummer: **0 241 661**
**B1**

(12) **EUROPÄISCHE PATENTSCHRIFT**

(45) Veröffentlichungstag der Patentschrift:
**14.12.88**

(21) Anmeldenummer: **87101900.6**

(22) Anmeldetag: **11.02.87**

(51) Int. Cl.⁴: **C 07 C 13/02,** C 07 C 7/148,
C 07 C 6/06

(54) Verfahren zur Herstellung von technisch reinem, chlorfreiem Cyclohexadecadien.

(30) Priorität: **14.04.86 DE 3612539**

(43) Veröffentlichungstag der Anmeldung:
**21.10.87 Patentblatt 87/43**

(45) Bekanntmachung des Hinweises auf die Patenterteilung:
**14.12.88 Patentblatt 88/50**

(84) Benannte Vertragsstaaten:
**BE CH DE FR GB IT LI NL**

(56) Entgegenhaltungen:
**EP-A-0 182 333**
**WO-A-84/04936**
**GB-A-1 105 565**
**US-A-3 439 056**
**US-A-3 764 622**

**THE JOURNAL OF ORGANIC CHEMISTRY, Band 36,
1971. B.D. MOOKHERJEE et al.: "Synthesis of
Racemic Muscone and Cyclopentadecanone
(Exaltone) from 1,9-Cyclohexadecadiene", Seiten
3266-3270**

(73) Patentinhaber: **HÜLS AKTIENGESELLSCHAFT,
Patentabteilung / PB 15 - Postfach 13 20, D-4370
Marl 1 (DE)**

(72) Erfinder: **Kaufhold, Manfred, Dr., Jasminweg 20,
D-4370 Marl (DE)**

**Beschreibung**

Die Erfindung betrifft ein Verfahren zur Herstellung von technisch reinem, chlorfreiem Cyclohexadecadien, wobei unter technisch rein ein Gehalt nach gaschromatographischer Analyse um 90 % bzw. höher und unter chlorfrei ein Chlorgehalt kleiner als 10 ppm verstanden wird. Produkte mit dieser Qualität sind für die technische Weiterverarbeitung geeignet. Cyclohexadecadien mit dieser Reinheit ist ein wertvoller Ausgangsstoff beispielsweise für die bekannten Moschusriechstoffe Cyclohexadecanon, Cyclohexadecenon und Cyclohexadecanolid und andere. Das eingesetzte Cyclohexadecadien erhält man bei der Metathese von Cycloocten.

In der Literatur sind bereits Vorschläge zu Synthesen von Moschusriechstoffen auf Basis von Cyclohexadecadien gemacht worden. So beschreibt Lawson G. Wideman (in Journal of Org. Chem. 33 No. 12 (1968) 4541) die Monoaddition von Borwasserstoff an Cyclohexadecadien und die Oxidation des Addukts zum ungesättigten Keton. Die Erzeugung und Reaktion des Borwasserstoffs ist technisch aufwendig und von dem Chemikalienverbrauch hergesehen sehr kostspielig, d. h. diese Reaktion kommt aus wirtschaftlichen Gründen für eine technische Realisierung nicht in Frage.

Bei einer von B. D. Mookherjee et al. vorgeschlagenen Synthese wird zunächst das Monoepoxid des Cyclohexadecadiens hergestellt, das mit Butyllithium in den entsprechenden ungesättigten Alkohol überführt wird. Andere weniger kostspielige Chemikalien erwiesen sich für diese Reaktion als ungeeignet. Die anschließende Reaktion des ungesättigten Ketons mit Methylmagnesiumbromid und die darauffolgende Hydrierung liefert Muscon (s. J. Org. Chem. Vol. 36 No. 22 (1971) 3266). Auch bei diesem Syntheseweg ist die technische Realisierung sehr aufwendig und die benötigten Chemikalien sind kostspielig.

Wünschenswert wäre ein Verfahren, bei dem man das rohe - aus der technischen Metathese des Cyclooctens stammende - Cyclohexadecadien verwenden kann und daraus durch katalytische Reaktionen beispielsweise Cyclohexadecanon herstellen könnte. Hierbei müßte man das Dien katalytisch zum Cyclohexadecan hydrieren und dieses mit Luft zum Keton oxidieren oder man könnte das Monoepoxid des Cyclohexadecadiens katalytisch zum gesättigten Epoxid hydrieren und dies katalytisch zum Keton isomerisieren.

Das Problem bei diesen Reaktionsfolgen ist die geringe Reinheit des rohen Cyclohexadecadiens, insbesondere sein relativ hoher Chlorgehalt von 200 bis 500 ppm. Chlorhaltige Produkte können in den üblichen technischen Apparaturen wegen Korrosion nicht verarbeitet werden.

Bei Hydrierungen mit diesem Cyclohexadecadien mit hohem Chlorgehalt wird der Katalysator geschädigt, seine Aktivität herabgesetzt und seine Lebensdauer verringert.

Dagegen sind die obengenannten - in der Literatur veröffentlichten - aufwendigen Reaktionen mit dem chlorhaltigen Dien problemlos durchführbar.

Es besteht daher ein großes Interesse an technisch einfach durchführbaren, katalytischen Verfahren zur Herstellung von Moschusriechstoffen auf Basis von Cyclohexadecadien, da der Moschusduft eine sehr universell begehrte Duftnote ist und üblicherweise als die animalische Note der Parfüme angesehen wird (DE-OS-2 111 753).

Ziel der Erfindung war deshalb die Reinigung und Entchlorierung des rohen technisch anfallenden Cyclohexadecadiens mittels preiswerter Chemikalien.

Die sich hieraus ergebende Aufgabe wird erfindungsgemäß entsprechend den Angaben der Patentansprüche gelöst.

Überraschenderweise erhält man ein technisch reines, chlorfreies, d. h. mit einem Chlorgehalt kleiner 10 ppm und nach GC-Analyse über 90 %-iges Cyclohexadecadien, wenn man folgende Reinigungsstufen mit dem Rohprodukt durchführt.

1. Abdestillieren der Leichtsleder und gegebenenfalls von Wasser aus dem aus der Metathese des Cyclooctens stammenden Cyclohexadecadiens.

2. Zugabe einer stark basischen Substanz, beispielsweise eine Lösung von Alkalialkoholat in dem entsprechenden Alkohol und Rühren bei erhöhter Temperatur unter eventuellem Abdestillieren des zugesetzten Lösemittels, wie z. B. des Alkohols.

3. Abkühlen, Abtrennen der entstandenen Salze, beispielsweise durch Waschen mit Wasser und Abtrennen der organischen Phase.

4. Fraktionierte Destillation der Ölschicht zur Gewinnung von $C_8$-, $C_{16}$- und $C_{24}$-Verbindungen.
($C_8$-Verbindungen sind Substanzen, deren Summenformel 8 Kohlenstoffatome aufweist).

Es zeigt sich nun überraschenderweise, daß nach der Reaktion mit der stark basischen Verbindung der Chlorgehalt von 200 bis 500 ppm zwar nur auf 50 bis 100 ppm gesenkt wurde, daß aber diese chlorhaltigen Reaktionsprodukte ohne Korrosionsgefahr in Apparaturen aus üblichen Materialien verarbeitet werden können und daß nach der fraktionierten Destillation die wertvollen $C_8$- und $C_{16}$-Verbindungen enthaltenden Destillationsschnitte chlorfrei sind, während die wertlosen Destillationsvor- und -zwischenläufe das Restchlor enthalten.

Dieser überraschende Befund ermöglicht also auf einfache Weise die Gewinnung von technisch reinem, chlorfreiem Cyclohexadecadien.

Um die Durchführung der einzelnen Stufen beschreiben zu können, sei folgende Zusammensetzung eines geeigneten Einsatzproduktes, eines aus der Metathese des Cyclooctens stammenden Cyclohexadecadien als Beilspiel genannt.

| $C_6$-Kohlenwasserstoffe | ca. | 20 % |
| Toluol | ca. | 5 % |
| Cycloocten | ca. | 1 % |
| Cyclooctan | ca. | 20 % |
| $C_{16}$-Kohlenwasserstoffe | ca. | 30 % |
| $C_{24}$-Kohlenwasserstoffe | ca. | 5 % |
| Nicht identifizierte Verbindungen | ca. | 19 % |
| | ca. | 100 % |

Der Chlorgehalt liegt bei 240 ppm.

Die praktische Durchführung des erfindungsgemäßen Verfahrens erfolgt in den folgenden Stufen:

1. Abdestillieren der $C_6$-Kohlenwasserstoffe und von suspendiertem und gelöstem Wasser bei Normaldruck bis zu einer Sumpftemperatur von 100 bis 170° C, vorzugsweise von 140 bis 150° C.

2. Zugabe einer stark basischen Substanz, beispielsweise einer Alkalialkoholat-Lösung in dem entsprechenden Alkohol bei diesen Temperaturen unter Rühren und eventuellem Abdestillieren des eingesetzten Alkohols. Als Alkalialkoholate sind vorzugsweise Natrium- und Kaliumalkoholat geeignet. Bevorzugt setzt man die Methylate, Ethylate, n-, iso- oder tert. -Butylate ein. Man setzt das Alkalialkoholat bevorzugt molmäßig, bezogen auf den Chlorgehalt im Einsatzprodukt in Mengen von 1 bis 100 Mol auf 1 g Atom Chlor, vorzugsweise 5 bis 50 Mol auf 1 g Atom Chlor zu. Die Behandlung mit dem Alkalialkoholat erfolgt im allgemeinen bei Temperaturen von 100 bis 250° C, bevorzugt von 120 bis 170° C, insbesondere von 140 bis 150° C.

3. Abkühlen auf 50 bis 0° C, vorzugsweise auf Raumtemperatur und Entfernung der entstandenen Salze und gegebenenfalls des überschüssigen Alkoholates beispielsweise durch eine Wasserwäsche. Die Wasserwäsche erfolgt im allgemeinen bei Temperaturen von 10 bis 50° C. Zur Wasserwäsche setzt man vorzugsweise 10 bis 100 % Wasser, bezogen auf das Einsatzprodukt, zu. Der Chlorgehalt liegt nach der Wäsche bei 50 bis 100 ppm.

4. Fraktionierte Destillation zur Gewinnung von chlorfreien $C_8$- und $C_{16}$-Destillationsschnitten.

Die erste Stufe wird vorzugsweise in Glas- oder Emailleapparaturen und die 2. bis 4. Stufe bevorzugt in den üblichen Stahlapparaturen durchgeführt, ohne daß Korrosionsgefahr besteht.

Wie bereits erwähnt, kann man das so erhaltene technisch reine, chlorfreie Cyclohexadecadien zur katalytischen Hydrierung oder zur Epoxidation einsetzen, um zu wertvollen, preiswerten Ausgangsstoffen zur Herstellung von Moschusriechstoffen zu gelangen.

Den folgenden Beispielen ist weiteres Zahlenmaterial zu entnehmen; sie sollen das erfindungsgemaße Verfahren näher erlautern:

**Beispiel 1**

Ein Produkt aus der Metathese des Cyclooctens mit folgender Zusammensetzung wird eingesetzt:

| $C_6$-Kohlenwasserstoffe | 22,3 % |
| Toluol | 3,5 % |
| Cycloocten | 0,8 % |
| Cyclooctan | 19,3 % |
| $C_{16}$-Kohlenwasserstoffe | 33,1 % |
| $C_{24}$-Kohlenwasserstoffe | 4,4 % |
| Nicht identifizierte Verbindungen | 16,6 % |
| | 100,0 % |

1 000 g dieses Produktgemisches werden bei Normaldruck an einer 0,5 m langen mit Multifil-Füllkörpern gefüllten Glaskolonne bei einem Verhältnis Rücklauf zur Abnahme wie 5 : 1 andestilliert, wobei die Sumpftemperatur von 104 auf 149° C erhöht wird und am Kopf sich Temperaturen von 62 bis 76° C einstellen.

Es fallen 209 g Destillat an, die verworfen werden. Der Destillationsrückstand zeigt folgende Zusammensetzung:

3

| | |
|---|---|
| C$_6$-Kohlenwasserstoffe | 0,9 % |
| Toluol | 4,8 % |
| Cycloocten | 1,0 % |
| Cyclooctan | 24,5 % |
| C$_{16}$-Kohlenwasserstoffe | 42,1 % |
| C$_{24}$-Kohlenwasserstoffe | 5,6 % |
| Nicht identifizierte Verbindungen | 21,1 % |
| | 100,0 % |

Der Chlorgehalt liegt bei 400 ppm.

Zur Dechlorierung wird eine Glasapparatur benutzt, die aus Dreihalskolben, Rührer, Thermometer und Destillationsvorrichtung besteht.

500 g des Destillationsrückstandes werden eingesetzt und unter Rühren am Rückfluß zum Sieden erhitzt, wobei sich eine Temperatur von 147°C einstellt. Dann werden 12 g einer 30,7 %-igen Natriummethylat-Methanol-Lösung innerhalb von 45 Minuten zugetropft und die Temperatur bei ca. 150°C gehalten. Hierbei destilliert Methanol ab, das verworfen wird.

Nach fünf Stunden wird auf Raumtemperatur abgekühlt und mit 300 g Wasser gewaschen. Danach liegt der Chlorgehalt bei 66 ppm. 500 g des in dieser Weise behandelten Produktes werden an einer 0,5 m langen mit Multifil-Füllkörpern gefüllten Glaskolonne fraktioniert destilliert. Die Chlorgehalte der erhaltenen Fraktionen sind in der rechten Spalte aufgeführt:

| Fr.Nr. | Temp.°C (am Kopf) | Gewicht g | Druck mbar | Verhältnis Rücklauf zur Abnahme | Chlorgehalt in ppm |
|---|---|---|---|---|---|
| 1 | 47 bis 83 | 110,2 | 133 | 5 : 1 | 110 |
| 2 | 83 bis 85 | 98,3 | 133 | 5 : 1 | 4,5 |
| 3 | 57 bis 158 | 85,2 | 13 | 5 : 1 | 1 000 |
| 4 | 158 bis 163 | 175,2 | 13 | 5 : 1 | 8 |
| 5 | 163 bis 202 | 8,0 | 13 | 5 : 1 | 280 |
| Rückstand | | 20,1 | | | |

Die Fraktion 2 besteht nach gaschromatographischer Analyse zu 3,9 % aus Cycloocten und zu 94,6 % aus Cyclooctan mit einem Chlorgehalt von nur 4,5 ppm.

Die gaschromatographische Analyse einer hydrierten Probe von der Fraktion 4 zeigt einen Gehalt an Cyclohexadecan von 97,4 %, d. h. die Fraktion 4 besteht zu ca. 97 % aus Cyclohexadecadienen. Die verschiedenen Isomeren können gaschromatographisch nicht zugeordnet werden. Der niedrige Chlorgehalt mit 8 ppm gestattet die technische Weiterverarbeitung wie beispielsweise eine katalytische Hydrierung in der üblichen Weise.

Die Durchführung des Korrosionstestes erfolgt mit dem Produkt, bei dem die C$_6$-Kohlenwasserstoffe abdestilliert sind, in der Weise, daß man die Behandlung mit Natriummethylat in Gegenwart von Spannbügelproben mit Schweißnaht aus austenitischem CrNiMo-Stahl 1,4571 vornimmt. Nach einer Testzeit von 256 Stunden zeigen die Probekörper keinen Korrosionsabtrag und keinen Korrosionsangriff.

In analoger Weise wird auf Korrosion während der Destillation geprüft. Nach einer Destillationsdauer von 598 Stunden ist an den Spannbügelproben aus Werkstoff 1,4571 kein Korrosionsangriff und kein Korrosionsabtrag feststellbar.

**Patentansprüche**

1. Verfahren zur Herstellung von technisch reinem, chlorfreiem Cyclohexadecadien durch Reinigung eines Produktgemisches, das bei der Metathese von Cyclooocten anfällt,
dadurch gekennzeichnet,
daß man zuerst die leichtsiedenden C$_6$-Kohlenwasserstoffe sowie gegebenenfalls vorhandenes gelöstes und/oder suspendiertes Wasser abdestilliert, dann das Sumpfprodukt mit einer Lösung einer stark basischen Substanz bei 100 bis 250°C behandelt, anschließend auf Temperaturen von 0 bis 50°C abkühlt und dann nach Abtrennung der entstandenen Salze das so vorbehandelte Produktgemisch in üblicher Weise fraktioniert destilliert.

2. Verfahren nach Anspruch 1,
dadurch gekennzeichnet,
daß man als stark basische Substanzen Alkalialkoholate, vorzugsweise Natrium- oder Kaliumalkoholate einsetzt.

3. Verfahren nach Anspruch 1 und 2,
dadurch gekennzeichnet,

daß man eine Lösung der Alkalialkoholate in dem entsprechenden Alkohol einsetzt.

4. Verfahren nach einem der Ansprüche 1 bis 3,
dadurch gekennzeichnet,
daß man die Behandlung mit den stark basischen Zusätzen bei Temperaturen von 120 bis 170°C, vorzugsweise 140 bis 150°C durchführt.

5. Verfahren nach einem der Ansprüche 1 bis 4,
dadurch gekennzeichnet,
daß man die Alkalialkoholate molmäßig, bezogen auf den Chlorgehalt des Einsatzproduktes in Mengen von 1 bis 100 Mol auf 1 g Atom Chlor, vorzugsweise 5 bis 50 Mol auf 1 g Atom Chlor einsetzt.

6. Verfahren nach einem der Ansprüche 1 bis 5,
dadurch gekennzeichnet,
daß man die entstandenen Salze durch eine Wasserwäsche entfernt.

7. Verfahren nach einem der Ansprüche 1 bis 6,
dadurch gekennzeichnet,
daß man bei der Wasserwäsche 10 bis 100 % Wasser, bezogen auf das Einsatzprodukt, zusetzt.

8. Verfahren nach einem der Ansprüche 1 bis 7,
dadurch gekennzeichnet,
daß man die Wasserwäsche bei Temperaturen von 10 bis 50°C durchführt.

## Claims

1. A process for production of technically pure, chlorine-free cyclohexadiene by purifying a product mixture arising from the metathesis of cycloctene, characterized in that first of all low boiling point $C_6$-hydrocarbons and any dissolved or suspended water which may be present are distilled off, then the bottom phase product is treated with a solution of a strongly basic substance at 100 to 250°C, then cooled to a temperature of 0 to 50°C, and then, after separation of the resulting salts, the product mixture which has been so treated is fractionally distilled in the usual way.

2. A process according to Claim 1, characterized in that an alkali alcoholate, preferably sodium or potassium alcoholate, is employed as the strongly basic substance.

3. A process according to Claim 1 and 2, characterized in that a solution of alkali alcoholate in the corresponding alcohol is employed.

4. A process according to one of Claims 1 to 3, characterized in that treatment with the strongly basic substance is performed at temperatures of 120 to 170°C, preferably 140 to 150°C.

5. A process according to one of Claims 1 to 4, characterized in that the alkali alcoholate is employed on a molar basis related to the chlorine content of the starting product in amounts of 1 to 100 mole to 1 g atom chlorine, preferably 5 to 50 mole to 1 g atom chlorine.

6. A process according to one of claims 1 to 5, characterized in that the salts produced are removed by a wash with water.

7. A process according to one of Claims 1 to 6, characterized in that in the water wash 10 to 100 % water, relative to the starting product, is added.

8. A process according to one of Claims 1 to 7, characterized in that the water wash is performed at temperatures of 10 to 50°C.

## Revendications

1. Procédé d'obtention de cyclohexadécadiène techniquement pur, dépourvu de chlore par purification d'un mélange de produit qui est obtenu par métathèse (double décomposition) du cyclo-octène caractérisé en ce que l'on sépare en premier lieu les hydrocarbures en $C_6$ qui bouillent facilement ainsi que l'eau éventuellement présente, dissoute et/ou mise en suspension par distillation, qu'ensuite on traite le produit de stockage avec une solution de substance fortement basique à 100 à 250°C qu'on refroidit après à des températures allant de 0 à 50°C et qu'ensuite après séparation des sels qui se sont formés, on soumet le mélange de produits ainsi prétraité à une distillation fractionnée de la manière usuelle.

2. Procédé selon la revendication 1, caractérisé en ce que l'on met en oeuvre comme substances fortement basiques des alcoolates de métal alcalin, de préférence des alcoolates de sodium ou de potassium.

3. Procédé selon les revendications 1 et 2, caractérisé en ce que l'on met en oeuvre une solution de l'alcoolate de métal alcalin dans l'alcool correspondant.

4. Procédé selon l'une des revendications 1 à 3, caractérisé en ce que l'on exécute le traitement avec les additifs fortement basiques à des températures allant de 120 à 170°C, de préférence 140 à 150°C.

5. Procédé selon l'une des revendications 1 à 4, caractérisé en ce que l'on met en oeuvre l'alcoolate de métal alcalin en proportion molaire, rapporté à la teneur en chlore du produit ajouté en quantités allant de 1 à 100 mol pour 1 atome g de chlore, de préférence de 5 à 50 mol pour 1 atome g de chlore.

6. Procédé selon l'une des revendications 1 à 5, caractérisé en ce que l'on élimine les sels qui se sont formés par des lavages à l'eau.

7. Procédé selon l'une des revendications 1 à 6, caractérisé en ce que l'on ajoute pour les lavages à l'eau de 10 à 100 % d'eau, rapporté au produit d'addition.

8. Procédé selon l'une des revendications 1 à 7, caractérisé en ce que l'on effectue les lavages à l'eau à des températures allant de 10 à 50° C.